# EUROPEAN PATENT APPLICATION

(11) **EP 3 312 287 A1**
(43) Date of publication of application: **25.04.2018**
(21) Application number: 16811739.8
(22) Date of filing: 17.06.2016
(51) Int. Cl.: C12Q 1/02, G01N 33/15, C07K 16/12, C12N 1/20, C12N 5/078, C12N 5/0787

(54) **METHOD FOR MEASURING COMPLEMENT-DEPENDENT BACTERICIDAL FUNCTION WITH RESPECT TO PNEUMOCOCCI**

(30) Priority: 18.06.2015 JP 2015122520
(71) Applicant: The Research Foundation for Microbial Diseases of Osaka University, Suita-shi, Osaka 565-0871 (JP); Osaka University, Suita-shi, Osaka 565-0871 (JP)
(72) Inventor: AKEDA, Yukihiro, Suita-shi Osaka 565-0871 (JP); MIYATAKE, Hiroshi, Kanonji-shi Kagawa 768-0065 (JP); PIAO, Zhenyu, Kanonji-shi Kagawa 768-0065 (JP); KOIZUMI, Yuka, Kanonji-shi Kagawa 768-0065 (JP)
(74) Representative: Brantsandpatents bvba
(86) International application number: PCT/JP2016/068069
(87) International publication number: WO 2016/204265

(57) **Abstract**

The present invention relates to a measurement method for complement-dependent bactericidal activity against *Streptococcus pneumoniae,* and provides a measurement method capable of measuring complement-dependent bactericidal activity against *Streptococcus pneumoniae* of any capsular serotype. Complement-dependent bactericidal activity against *Streptococcus pneumoniae* is measured using capsule-deficient *Streptococcus pneumoniae,* that is, non-encapsulated or substantially non-encapsulated, or transparent *Streptococcus pneumoniae.* The measurement of the complement-dependent bactericidal activity against *Streptococcus pneumoniae* of any capsular serotype is enabled.

## Description

### Technical Field

The present invention relates to a measurement method for complement-dependent bactericidal activity against *Streptococcus pneumoniae.*

The present application claims priority from Japanese Patent Application No. 2015-122520, which is incorporated herein by reference.

### Background Art

*Streptococcus pneumoniae* is a major bacterial respiratory pathogen, and is isolated with high frequency as a bacterium causing pneumonia. *Streptococcus pneumoniae* causes invasive pneumococcal disease (IPD) including meningitis and sepsis, community-acquired pneumonia (CAP), and nursing and healthcare associated pneumonia (NHCAP) in children and adults infected with the bacterium. *Streptococcus pneumoniae* has a capsule formed of a polysaccharide important for pathogenicity on an outside of its cell, and its capsular serotypes are classified into a total of 118 or more types including 93 or more types belonging to 21 groups and 25 single types. The capsule serves as a barrier, and hence this bacterium exhibits strong resistance to a phagocytic action of human polymorphonuclear leukocytes.

The current pneumococcal vaccines contain as antigens purified capsular polysaccharides, which determine serotypes of *Streptococcus pneumoniae.* A heptavalent pneumococcal conjugate vaccine (PCV7) containing a non-toxic mutant of diphtheria toxin (CRM₁₉₇) conjugated with antigens purified from seven capsular serotypes of *Streptococcus pneumoniae* that were liable to cause severe infectious diseases in children and had a high rate of resistance was sold in the market. After introduction of PCV7, the incidence of invasive pneumococcal disease caused by the seven capsular serotypes of *Streptococcus pneumoniae* covered by this vaccine was clearly reduced, but an increase in the incidence of pediatric and adult IPD caused by nonvaccine serotypes, such as serotype 19A, became a problem. For this reason, a 13-valent pneumococcal conjugate vaccine (PCV13) containing PCV7 and additional capsular polysaccharide antigens of six other serotypes was introduced, and was already approved and sold in the market. In addition, a 23-valent pneumococcal capsular polysaccharide vaccine (PPV23) for adults containing 23 types of purified capsular antigens was sold in the market.

Croney et al. performed a large-scale survey on pediatric invasive pneumococcal isolates collected in Alabama in the United States between 2002 and 2010 before PCV13 vaccination, and as a result, reported that only 60% of these isolates included serotypes covered by PCV13 and the remaining 40% thereof included 17 serotypes that were not covered by PCV13. Also in Japan, publicly-aided pediatric PCV7 vaccination was started in 2011, but it was reported in 2012 that the incidence of IPD caused by nonvaccine serotypes was increased as in the United States. It is unreal to continue supplementing the current vaccines with capsular polysaccharide antigens of nonvaccine serotypes. This implies limitations of the current pneumococcal vaccines based on capsular polysaccharides.

Pneumococcal surface protein A (PspA), which is a pneumococcal surface protein antigen, has drawn attention as a novel pneumococcal vaccine antigen to compensate for the above-mentioned drawback of the current pneumococcal vaccines. α-Helix and proline-rich regions of PspA are known to have antigen epitopes for recognition by antibodies exhibiting protective actions against pneumococcal infection. According to amino acid sequences of clade definition regions present in its N terminus, PspA is roughly classified into three families, which are further classified into six subgroups called clades. Regarding PspA family distribution, families 1 and 2 account for 98% or more of pneumococcal clinical isolates. PspA is reported to also serve as a virulence factor to inhibit deposition of complement C₃ onto surfaces of pneumococcal cells. An anti-PspA-specific antibody is reported to exhibit protective activity against pneumococcal infection by antagonizing inhibitory activity of PspA against complement deposition. This infection protective activity is also exhibited by antibodies that cross-recognize different families of PspAs. Due to diversity of cross-reactive immunogenicity among different PspAs, appropriate selection of a combination of clades belonging to family 1 and family 2 exhibiting broader cross-reactive immunogenicity is considered to be important in development of PspA-based vaccines.

Cholin binding protein A (CbpA) and pneumococcal surface adhesion A (PsaA) are known as proteins expressed on pneumococcal cell surfaces and also known as pathogenic factors, and hence are considered as vaccine antigen candidates. CbpA is a choline binding protein as with PspA, and is detected in about 75% of all pneumococcal strains. When CbpA binds to an immunoglobulin receptor on an epithelial cell or mucous membrane cell surface, *Streptococcus pneumoniae* is taken up into cells and then undergoes transcytosis to enter a living body (Non Patent Literature 1). PsaA is a protein present in all serotypes of *Streptococcus pneumoniae,* and is a factor important for adhesion to host cells as with any other bacterial adhesion molecule (adhesin) (Non Patent Literature 2).

Measurement of a serotype-specific IgG concentration by an ELISA method and measurement of serotype-specific opsonic activity by multiplex opsonization assay (MOPA) may be performed for the purpose of evaluating immunity induction activity (immunogenicity) of a pneumococcal vaccine or humoral immunity of a patient suffering from pneumococcal infectious disease. The measurement of the serotype-specific IgG concentration by the ELISA method may be performed by quantification based on an amount of an IgG antibody binding to a CPS antigen of each serotype immobilized on an ELISA plate after absorption of antibodies against common antigens of *Streptococcus pneumoniae* in serum [cell wall polysaccharide and 22F capsular polysaccharide (CPS)] (Non Patent Literature 3). At present, a serotype-specific IgG concentration of ≥0.20 µg/ml (third-generation ELISA) is shown as a protection threshold for group infection of a heptavalent pneumococcal conjugate vaccine (PCV7) for children against invasive pneumococcal disease (IPD) in children (Non Patent Literature 4). Meanwhile, Burton et al. developed a MOPA method allowing measurement of opsonic activity (opsonization index; OI) against each of a plurality of serotypes for evaluating a function of a serotype-specific antibody after pneumococcal vaccination (Non Patent Literature 5). However, all the evaluation methods are measurement methods specialized in capsular serotypes.

### Citation List

### Non Patent Literature

[NPL 1] Mol Microbiol 25: 819-829, 1997
[NPL 2] Microb Pathog 21: 17-22, 1996
[NPL 3] Clin Diagn Lab Immunol 8: 266-272, 2001
[NPL 4] Clin Vaccine Immunol 18: 2161-2167, 2011
[NPL 5] Clin Vaccine Immunol 13: 1004-1009, 2006

### Summary of Invention

### Technical Problem

The present invention relates to a measurement method for complement-dependent bactericidal activity against *Streptococcus pneumoniae.* It is an object of the present invention to provide a measurement method capable of measuring complement-dependent bactericidal activity against *Streptococcus pneumoniae* of any capsular serotype.

### Solution to Problem

The inventors of the present invention have made extensive investigations, and as a result, have found that the object of the present invention is achieved by measuring complement-dependent bactericidal activity (opsonic activity: OPA) against *Streptococcus pneumoniae* using capsule-deficient *Streptococcus pneumoniae,* that is, non-encapsulated or substantially non-encapsulated, or transparent *Streptococcus pneumoniae.* The above-mentioned method enables the measurement of the complement-dependent bactericidal activity against *Streptococcus pneumoniae* of any capsular serotype.

That is, the present invention includes the following.
1. A measurement method for complement-dependent bactericidal activity of a pneumococcal-specific antibody against *Streptococcus pneumoniae,* the measurement method including the following steps 1) to 3):
   1) mixing capsule-deficient *Streptococcus pneumoniae* (a) and a sample (b) containing a pneumococcal-specific antibody;
   2) further adding, after the step 1), a complement component (c) and a phagocyte (d); and
   3) determining, after the step 2), a concentration of the sample (b) at which a certain amount of the capsule-deficient *Streptococcus pneumoniae* (a) is killed.
2. A measurement method for complement-dependent bactericidal activity according to Item 1, in which the certain amount in the step 3) is 50%.
3. A measurement method for complement-dependent bactericidal activity according to Item 1 or 2, in which a usage amount of the complement component (c) in the step 1) is from 1/400 to 1/100 with respect to that of a complement component (c') to be used in a case of measurement using encapsulated *Streptococcus pneumoniae* (a').
4. A measurement method for complement-dependent bactericidal activity according to any one of Items 1 to 3, in which the capsule-deficient *Streptococcus pneumoniae* as defined in each of the steps 1) and 2) includes non-encapsulated *Streptococcus pneumoniae* or transparent *Streptococcus pneumoniae.*
5. A measurement method for complement-dependent bactericidal activity according to any one of Items 1 to 4, in which the capsule-deficient *Streptococcus pneumoniae* (a) includes capsule-deficient *Streptococcus pneumoniae* derived from any one selected from pneumococcal strains WU2, D39, KK1162, ATCC6303, BG9739, TIGR4, and EF5668.
6. A measurement method for complement-dependent bactericidal activity according to any one of Items 1 to 5, in which the phagocyte (d) in the step 1) includes an HL-60-derived cell.
7. A measurement method for complement-dependent bactericidal activity according to any one of Items 1 to 6, in which the sample (b) includes a blood sample collected from a test subject.
8. A measurement method for complement-dependent bactericidal activity according to Item 7, in which the sample (b) includes a blood sample collected from a test subject vaccinated with a pneumococcal vaccine.
9. An examination method for immunogenicity of a pneumococcal vaccine, the examination method including performing an examination based on a result measured by the measurement method of any one of Items 1 to 8.

### Advantageous Effects of Invention

According to the measurement method for complement-dependent bactericidal activity of the present invention, a complement-dependent bactericidal effect can be easily and effectively investigated irrespective of capsular serotypes of infecting *Streptococcus pneumoniae.* In addition, opsonic activity measured by the method of the present invention can be utilized as a surrogate marker for a vaccine using a pneumococcal surface protein. This enables the provision of a certain evaluation for the effectiveness of the vaccine using a pneumococcal surface protein irrespective of capsular serotypes of *Streptococcus pneumoniae.*

### Brief Description of Drawings

FIG. 1 is a diagram for illustrating one mode of a measurement system of the present invention (Example 1).
FIG. 2 is a graph for showing the opsonic activity of anti-PspA clade 2 mouse immune serum or nonimmune serum against pneumococcal strain R6 (Example 1).
FIG. 3 is a graph for showing the opsonic activity of anti-PspA clade 2 mouse immune serum or anti-PspA clade 3 mouse immune serum against pneumococcal strain R6 (Example 1).
FIG. 4 is a graph for showing the opsonic activities of IgG fraction and anti-PspA IgG fraction purified from human plasma against pneumococcal strain R6 (Example 2).
FIG. 5 is a graph for showing the measurement results of amounts of anti-PspA IgG contained in IgG fraction and anti-PspA IgG fraction purified from human plasma (Test Example).
FIG. 6 is a graph for showing the measurement results of amounts of anti-capsular (serotype 3) IgG, anti-CbpA IgG, and anti-PsaA IgG contained in IgG fraction and anti-PspA IgG fraction purified from human plasma (Test Example).

### Description of Embodiments

The present invention relates to a measurement method for complement-dependent bactericidal activity of a pneumococcal-specific antibody against *Streptococcus pneumoniae,* the measurement method including the following steps 1) to 3):
1) mixing capsule-deficient *Streptococcus pneumoniae* (a) and a sample (b) containing a pneumococcal-specific antibody;
2) further adding, after the step 1), a complement component (c) and a phagocyte (d); and
3) determining, after the step 2), a concentration of the sample (b) at which a certain amount of the capsule-deficient *Streptococcus pneumoniae* (a) is killed.

The above-mentioned measurement method relates to a modified method of the so-called opsonophagocytic killing assay (OPA).

Herein, the "capsule-deficient *Streptococcus pneumoniae* (a)" refers to *Streptococcus pneumoniae* having no capsule or having a capsule thinner than that of general encapsulated *Streptococcus pneumoniae,* and examples thereof include non-encapsulated *Streptococcus pneumoniae* and transparent *Streptococcus pneumoniae.* In this case, the capsule may be completely deficient, or the capsule may be present as long as a surface protein is sufficiently exposed. The capsule-deficient *Streptococcus pneumoniae* may be artificially produced capsule-deficient *Streptococcus pneumoniae,* or may be naturally occurring capsule-deficient *Streptococcus pneumoniae.* The non-encapsulated *Streptococcus pneumoniae* may be artificially produced by artificial deletion of a group of genes involved in capsule synthesis, and antibiotic addition. The transparent *Streptococcus pneumoniae* may be produced by a method involving adding a chemical, for example, xylitol, and a capsule-degrading enzyme into a culture system, and shaking culture or the like. An example of the non-encapsulated *Streptococcus pneumoniae* may be pneumococcal strain R6. In addition, in order to artificially produce the capsule-deficient *Streptococcus pneumoniae,* for example, a capsular serotype 2 strain D39, capsular serotype 3 strains WU2, KK1162, and ATCC6303, and capsular serotype 4 strains BG9739, TIGR4, and EF5668 may be used as strains from which the capsule-deficient *Streptococcus pneumoniae* is derived.

Herein, regarding the certain amount of the capsule-deficient *Streptococcus pneumoniae* (a), the "certain amount" only needs to be a certain amount specified within the range of from 0% to 100% of the amount of the capsule-deficient *Streptococcus pneumoniae* added to a measurement system for complement-dependent bactericidal activity, and is not particularly limited. Specifically, for example, the "certain amount" is a bacterial amount specified as follows: 60%, 50%, 40%, 30%, or 20% of the capsule-deficient *Streptococcus pneumoniae* (a) of the present invention. In particular, 50% or 60% is suitably applied. In the measurement method for complement-dependent bactericidal activity of the present invention, the complement-dependent bactericidal activity may be measured by measuring the concentration of a sample at which 60%, 50%, 40%, 30%, or 20% of the capsule-deficient *Streptococcus pneumoniae* (a), particularly suitably 50% or 60% thereof is killed.

Herein, the "sample (b)" refers to a sample to be applied to the measurement method for complement-dependent bactericidal activity of the present invention, and specifically refers to a sample to be used for the measurement of opsonic activity. For the measurement of the present invention, the sample only needs to be a sample that may contain a pneumococcal-specific antibody, and is not particularly limited. Examples of such sample that may contain a pneumococcal-specific antibody may include a blood sample collected from a test subject to which a pneumococcal vaccine has been administered, and a blood sample collected from a test subject suspected of infection with *Streptococcus pneumoniae.* Herein, the blood sample only needs to be a blood-derived sample, and may be serum or plasma. For example, the sample may be a sample that may be used in a general clinical examination to be used after the treatment of blood collected in a clinical examination. Herein, the test subject only needs to be a vertebrate, for example, a human or a mouse, and is particularly suitably a human. According to the present invention, the pneumococcal-specific antibody in the sample can be measured for its complement-dependent bactericidal activity by subjecting the pneumococcal-specific antibody in the sample and a complement to a reaction.

Herein, the "complement component (c)" that may be used only needs to be a complement that may be used in the measurement of the complement-dependent bactericidal activity of the pneumococcal-specific antibody against *Streptococcus pneumoniae.* A commercially available complement reagent may be used as long as the complement reagent is such complement as described above. For example, in accordance with the protocol of Protocol for a multiplexed opsonophagocytic killing assay (UAB-MOPA, reference 2) for antibodies against Streptococcus pneumoniae Version E.02, December 2014 (www.vaccine.uab.edu), a complement component shown on page 16 may be used. The usage concentration of the complement is as follows: when the concentration of a complement component (c') to be used in the case of measurement using encapsulated (opaque) *Streptococcus pneumoniae* (a') is defined as 1, the usage amount of the complement component (c) in the step 1) may be set to from 1/400 to 1/100, preferably 3/400 with respect to the above-mentioned concentration. Specifically, the usage amount may be set to from 1/400 to 1/100, preferably 3/400 with respect to a usage concentration shown in Pelfreez 3-4 week baby rabbit complement, sterile (manufactured by Pelfreez).

Herein, the phagocyte (d) only needs to be a phagocyte applicable to the measurement of the complement-dependent bactericidal activity, and is not particularly limited. An example of the phagocyte to be generally used is, but not limited to, a human leukemia-derived HL-60-derived cell having induced phagocytic activity.

The measurement method for complement-dependent bactericidal activity of a pneumococcal-specific antibody against *Streptococcus pneumoniae* of the present invention enables the examination of immunogenicity of a pneumococcal vaccine. Herein, the "immunogenicity of a pneumococcal vaccine" refers to immunity induction activity, such as a property of inducing antibody production or cellular immunity, of a pneumococcal vaccine, and may be opsonic activity (opsonization index; OI). The opsonic activity measured by the method of the present invention can be utilized as a surrogate marker for a vaccine.

The "pneumococcal vaccine" applicable to the measurement method for complement-dependent bactericidal activity of the present invention may be a "pneumococcal vaccine" known per se, or may be any "pneumococcal vaccine" to be developed in the future. For example, a pneumococcal vaccine disclosed in WO 2014/045621 A1 may be used, and a pneumococcal vaccine newly targeting a surface protein to be developed in the future can be evaluated.

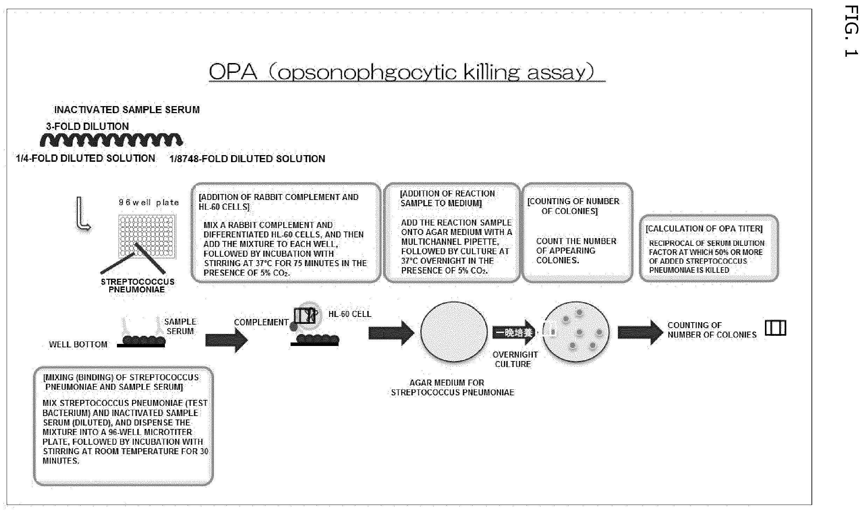

One mode of the measurement of complement-dependent bactericidal activity of a pneumococcal-specific antibody against *Streptococcus pneumoniae* (opsonophagocytic killing assay (OPA)) of the present invention is as illustrated in FIG. 1. For example, the measurement may be performed by a method including the following steps:
1) adding a sample (b) containing a pneumococcal-specific antibody to each well of a microplate in which capsule-deficient *Streptococcus pneumoniae* (a) has been seeded, followed by incubation;
2-1) further adding, after the step 1), a complement component (c) and a phagocyte (d), followed by culture;
2-2) adding the reaction solution of the step 2-1) to agar medium, followed by further culture; and
3) determining a concentration of the sample (b) at which a certain amount of the capsule-deficient *Streptococcus pneumoniae* (a) is killed, by counting the number of colonies of the capsule-deficient *Streptococcus pneumoniae* (a) appearing in the agar medium.

In the foregoing, the incubation in the step 1) may be performed under the conditions of room temperature, for example, from 10°C to 35°C and from 15 minutes to 60 minutes, preferably 30 minutes. The culture in the step 2-1) may be performed under the conditions of 37±1°C and from 30 minutes to 180 minutes, preferably from 40 minutes to 80 minutes, more preferably from 45 minutes to 75 minutes in the presence of 5% CO₂. Further, the culture in the step 2-2) may be performed under the conditions of 37±1°C and from 6 hours to 48 hours, preferably from 6 hours to 24 hours, more preferably 16 hours in the presence of 5% CO₂. Optimum conditions in a measurement system may be appropriately selected as the above-mentioned conditions.

A buffer or a medium known per se, or an applicable buffer or medium to be developed in the future may be used as a buffer or a medium to be used in the measurement of the complement-dependent bactericidal activity of the present invention without any particular limitation. Specifically, the measurement may be performed in accordance with the protocol of Protocol for a multiplexed opsonophagocytic killing assay (UAB-MOPA, reference 2) for antibodies against Streptococcus pneumoniae Version E.02, December 2014 (www.vaccine.uab.edu) except that matters specified in the present invention are applied as the capsule-deficient *Streptococcus pneumoniae* (a) to be used and the addition amount of the complement component (c).

### Examples

To help understanding of the present invention, the present invention is specifically described below by way of Examples, but it goes without saying that the present invention is not limited to these Examples.

### (Example 1) Opsonic Activity of Mouse Immune Serum

The opsonic activity (OPA) of each of anti-PspA clade 2 mouse immune serum or anti-PspA clade 3 mouse immune serum was measured by the following method (see FIG. 1). The anti-PspA clade 2 mouse immune serum or the anti-PspA clade 3 mouse immune serum was used as a sample.
(a) Capsule-deficient *Streptococcus pneumoniae*: non-encapsulated pneumococcal strain R6
(b) Sample: Anti-PspA clade 2 serum is serum obtained from a mouse immunized with PspA clade 2 protein disclosed in paragraph 0065 of WO 2014/045621 A1 serving as an antigen, and anti-PspA clade 3 serum is serum obtained from a mouse immunized with PspA clade 3 disclosed in paragraph 0065 of WO 2014/045621 A1 serving as an antigen. C57/BL6j mice (6- to 8-week-old, female) were subcutaneously inoculated with a mixture of 0.1 µg of PspA clade 2 or PspA clade 3 serving as an antigen, and 2.5 µg of CpGK3 and 5 µg of Alum serving as adjuvants. The inoculation was performed a total of three times every week, and whole blood was collected 1 week after the final immunization. Thus, immune serum was obtained.
(c) Complement component: Pelfreez 3-4 week baby rabbit complement, sterile (manufactured by Pelfreez)
(d) Phagocytes: Human leukemia-derived HL-60 cells were cultured in CM1 medium (500 ml of RPMI 1640, 50 ml of inactivated FBS, 5 ml of GlutaMax-1, and 5 ml of penicillin/streptomycin) at 37°C in the presence of 5% CO₂ and then the medium was exchanged for a medium for differentiation (500 ml of RPMI 1640, 50 ml of inactivated FBS, 5 ml of GlutaMax-1, and 4.55 ml of dimethyl formamide (DMF)) to perform differentiation induction. 4 Days to 5 days after the start of the culture, the HL-60 cells were washed with HBSS (without Ca⁺⁺, Mg⁺⁺) and then washed with HBSS (with Ca⁺⁺, Mg⁺⁺). The resultant cells were suspended at 1×10⁷ cells/ml in OBB solution (32 ml of distilled water, 4 ml of 10x HBSS (with Ca⁺⁺, Mg⁺⁺), 4 ml of 1% gelatin, and 2.12 ml of inactivated FBS) before use.

1) 20 µl of the sample (b) was added to each well of a 96-well microplate in which 500 CFU of the pneumococcal strain R6 (a) had been seeded, followed by incubation at room temperature for 30 minutes.
2-1) After the step 1), 10 µl (final concentration: 0.75%) of the complement component (c) was added, and the phagocytes (d) were added at 4×10⁵ cells, followed by incubation at 37°C for 75 minutes in the presence of 5% CO₂.
2-2) The reaction solution of the step 2-1) was added to 10 µl of agar medium, followed by culture at 37°C for 16 hours in the presence of 5% CO₂.
3) OPA activity was measured by counting the number of colonies of the pneumococcal strain R6 appearing in the agar medium to calculate a concentration of the sample (b) at which 50% of the pneumococcal strain R6 (a) was killed.

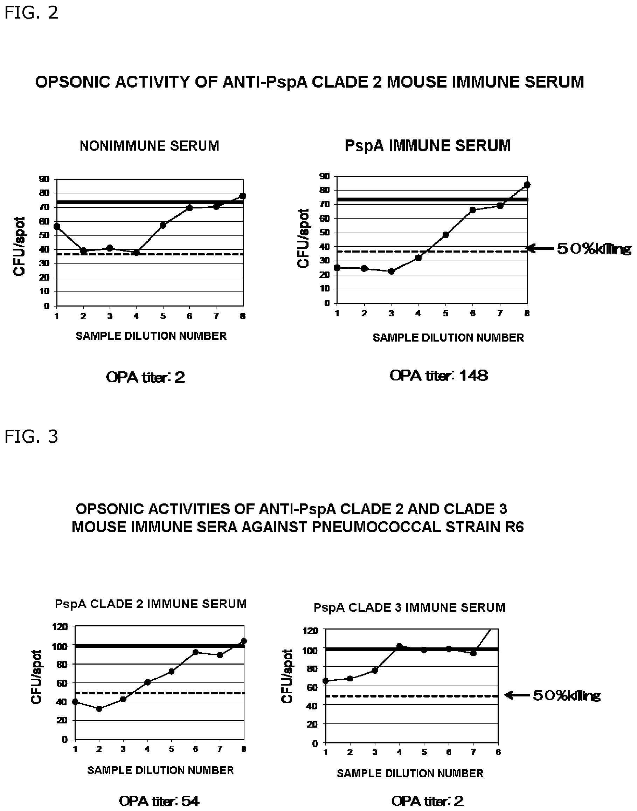

The results are shown in FIG. 2 and FIG. 3. In FIG. 2, the opsonic activity was investigated using the anti-PspA clade 2 mouse immune serum obtained in the (b). As a result, the opsonic activity of the immune serum against the test bacterium was found to be 70 times or more with respect to that of nonimmune serum. In FIG. 3, the opsonic activity of the anti-PspA mouse immune serum (PspA clade 2 or clade 3) against the test bacterium (pneumococcal strain R6, PspA clade 2) was investigated. As a result, the anti-PspA clade 2 mouse immune serum exhibited antigen-specific opsonic activity against the test bacterial strain, whereas the anti-PspA clade 3 mouse immune serum could not exhibit any opsonic activity, revealing that this test allowed the antigen-specific opsonic activity to be measured.

### (Example 2) Opsonic Activity using Human Anti-PspA IgG

In this Example, the opsonic activity of a sample containing human anti-PspA IgG was measured. The opsonic activity (OPA) was measured by the following method using as the sample (b) anti-PspA IgG fraction purified from human plasma by affinity chromatography using PspA clade 2 protein.

(a) Capsule-deficient *Streptococcus pneumoniae*: non-encapsulated pneumococcal strain R6 (the same as in Example 1)
(b) Sample: A human plasma sample containing human anti-PspA clade 2 antibody. The sample is used in the measurement of the opsonic activity. A production method for the sample to be used in this Example is as described below.
   Human plasma stored at -80°C was thawed at normal temperature, and total IgG contained in the plasma was purified using HiTrap rProtein G FF Column (manufactured by GE Healthcare) in accordance with the use procedure of the column. In addition, PspA clade 2 protein disclosed in paragraph 0065 of WO 2014/045621 A1 was immobilized on NHS-activated HP Column (manufactured by GE Healthcare), and then anti-PspA IgG in the human plasma was purified in accordance with the use procedure of the column.
(c) Complement component: Pelfreez 3-4 week baby rabbit complement, sterile (manufactured by Pelfreez)
(d) Phagocytes: The human leukemia-derived HL-60 cells shown in Example 1 were used as phagocytes.
(e) OPA test method: A test was performed by the same technique as in Example 1.

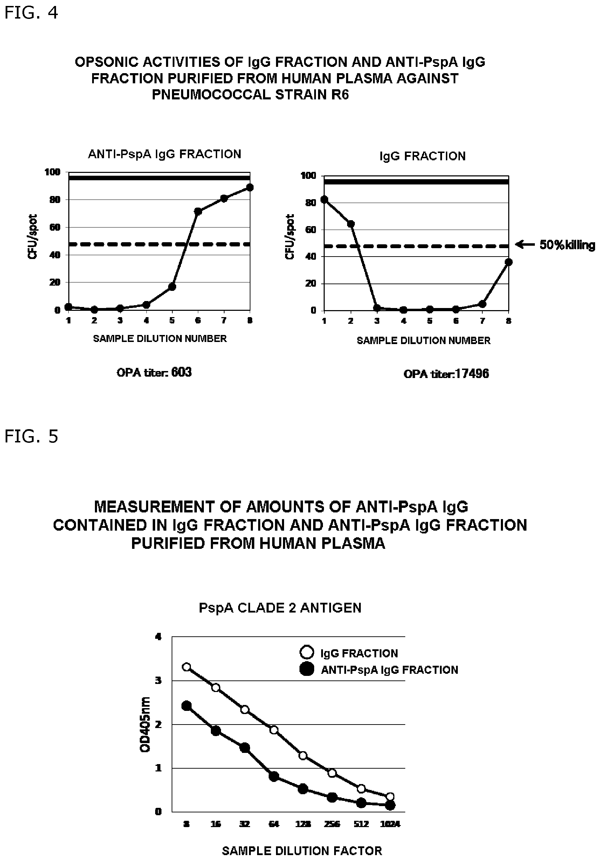

As a result of the measurement of the opsonic activity against the strain R6 using the purified human anti-PspA clade 2 IgG fraction and the purified total IgG fraction, the opsonic activity of the total IgG fraction against the pneumococcal strain R6 was 17,496. The opsonic activity of the purified anti-PspA IgG fraction was 603 (FIG. 4). The results revealed that the human anti-PspA IgG exhibited high opsonic activity against the pneumococcal strain R6. This is probably because opsonization was induced by the binding of the human PspA-specific IgG to PspA present in the pneumococcal strain R6. This suggests that the human anti-PspA-specific IgG may exhibit opsonic activity on any of the capsule-deficient pneumococcal strain and the transparent pneumococcal strain.

### (Test Example) Analysis of Sample (b)

In this Test Example, the amount of antigen-specific IgG against PsaA, CbpA, capsule (serotype 3), or PspA contained in the sample (b) produced in Example 2, that is, the purified human anti-PspA clade 2 IgG fraction was measured by an ELISA method.

### 1) Production of Antigens to be used in ELISA Method

PsaA and CbpA antigens were produced by a genetic recombination technique using *E. coli* DH5α Competent Cells (manufactured by TOYOBO) on the basis of the following method. A procedure for cloning of PsaA or CbpA was performed using *E. coli* DH5a Competent Cells (manufactured by TOYOBO). *E. coli* DH5α was cultured using Luria-Bertani (LB) or LB agar medium containing 30 µg/ml of kanamycin.
- PsaA was amplified by a PCR method using a primer set set forth in SEQ ID NOS: 1 and 2 and using genomic DNA of pneumococcal strain D39 as a template.
   PsaA-F (NdeI): GGAATTCCATATGGCTAGCGGAAAAAAAG (SEQ ID NO: 1)
   PsaA-R (HingIII): CCCAAGCTTTTATTTTGCCAATCCTTCAG (SEQ ID NO: 2)
- CbpA was amplified by a PCR method using a primer set set forth in SEQ ID NOS: 3 and 4 and using genomic DNA of pneumococcal strain D39 as a template. Gene portions encoding α-helix region (SEQ ID NO: 3) and prolin-rich region (SEQ ID NO: 4) on the N-terminal side of CbpA were used as primers.
   CbpA-F (NdeI): GGAATTCCATATGACAGAGAACGAGGGAAGTACCCA (SEQ ID NO: 3)
   CbpA-R (HingIII): CCCAAGCTTTTACCACATACCGTTTTCTTGTTTC (SEQ ID NO: 4)

### 2) Expression and Production of Antigens:

PCR products of PsaA and CbpA having restriction enzyme NdeI recognition sequence added to the N-terminal side and having HindIII recognition sequence added to the C-terminal side were inserted to restriction enzyme NdeI and HindIII recognition sites of a pET28a(+) vector. Thus, a plasmid expressing PsaA or CbpA protein was produced. The two gene fragments of PsaA and CbpA were identified for their sequences by sequencing. A CbpA- or PsaA-expressing plasmid was transformed into *E. coli* BL21 (DE3) to express a large amount of each protein. In order to purify each protein, *E. coli* BL21 (DE3) expressing the protein was subjected to shaking culture at 37°C in LB medium containing 30 µg/ml kanamycin. When the optical density (OD) at 600 nm reached about 0.8, 0.5 mM isopropyl-β-D-thiogalactopyranoside (IPTG) was added, and shaking culture was performed for an additional 3 hours. After that, the cells were collected. PsaA or CbpA protein was purified as a protein having a His-tag added to the N-terminal by Ni²⁺ affinity chromatography and a gel filtration method. The purified protein was identified using SDS-PAGE and a Westen blotting method.

### 3) Measurement of PsaA-, CbpA-, and PspA-specific IgG Antibodies

The amounts of antigen-specific IgG antibodies against PsaA, CbpA, capsular, and PspA antigens were measured by an ELISA method.

1 µg/ml PsaA, CbpA, and PspA antigens were added to a 96-well microplate at 100 µl/well, followed by incubation at 4°C overnight. The plate coated with the antigen was washed with phosphate buffered saline containing 0.05% Tween 20 (PBST). After that, total IgG fraction and purified PspA clade 2-specific IgG fraction purified from human plasma subjected to serial dilution were added to the wells at 50 µl/well, followed by incubation at 37°C for 30 minutes. After that, the microplate was washed with PBST, and 100 µl of alkaline phosphatase conjugated affini pure goat anti-human IgG diluted 5,000-fold was added to each well, followed by incubation at 37°C for 30 minutes. After that, the microplate was washed with PBST, and 100 µl of a substrate [4-nitrophenyl phosphate disodium salt hexahydrate diluted with substrate buffer (1 M diethanolamine, 0.5 mM MaCl₂)] was added to each well, followed by incubation at room temperature for 45 minutes under light shielding. After that, an optical density (OD) at 405 nm was measured.

### 4) Measurement of Serotype 3 Capsule-specific IgG Antibody

A serotype 3 capsular antigen (ATCC, 169-X) was prepared at 5 µg/ml, and then 100 µl thereof was added to each well of a 96-well microplate, followed by incubation at 37°C for 5 hours. The plate coated with the antigen was washed with PBST, and total IgG fraction and purified anti-PspA IgG fraction purified from human plasma subjected to serial dilution were added to the wells at 100 µl/well, followed by incubation at 37°C for 1 hour. After that, the microplate was washed with PBST, and then alkaline phosphatase conjugated affini pure goat anti-human IgG diluted 5,000-fold was added to each well at 100 µl/well, followed by incubation at 37°C for 1 hour. After that, the microplate was washed with PBST, and 100 µl of a substrate [4-nitrophenyl phosphate disodium salt hexahydrate diluted with substrate buffer (1 M diethanolamine, 0.5 mM MgCl₂)] was added to each well, followed by culture at 37°C under light shielding for 45 minutes. After the culture, an optical density (OD) at 405 nm was measured.

### 5) Results

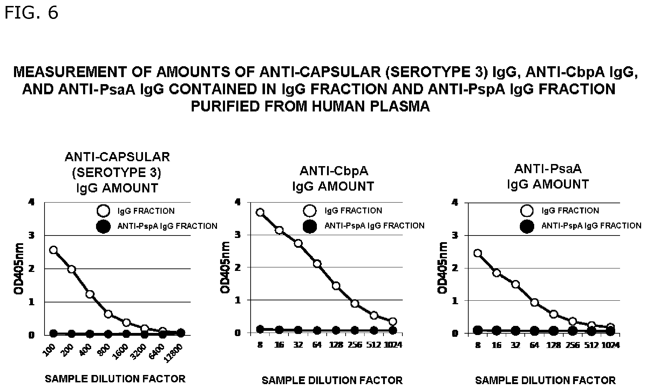

The anti-PspA IgG antibody was present at a high concentration in the anti-PspA IgG fraction purified from human plasma (FIG. 5). It was found that the anti-capsular (serotype 3) IgG, anti-CbpA IgG, and anti-PsaA IgG antibodies serving as antibodies against antigens derived from *Streptococcus pneumoniae* did not remain in the anti-PspA IgG fraction purified from human plasma (FIG. 6).

### Industrial Applicability

As described in detail above, according to the measurement method for complement-dependent bactericidal activity of the present invention, a complement-dependent bactericidal effect can be easily and effectively investigated irrespective of capsular serotypes of *Streptococcus pneumoniae.* In addition, opsonic activity measured by the method of the present invention can be utilized as a surrogate marker for a vaccine using a pneumococcal surface protein. This enables the provision of a certain evaluation for the effectiveness of the vaccine using a pneumococcal surface protein irrespective of capsular serotypes of *Streptococcus pneumoniae.*

## Claims

1. A measurement method for complement-dependent bactericidal activity of a pneumococcal-specific antibody against *Streptococcus pneumoniae,* the measurement method comprising the following steps 1) to 3):
1) mixing capsule-deficient *Streptococcus pneumoniae* (a) and a sample (b) containing a pneumococcal-specific antibody;
2) further adding, after the step 1), a complement component (c) and a phagocyte (d); and
3) determining, after the step 2), a concentration of the sample (b) at which a certain amount of the capsule-deficient *Streptococcus pneumoniae* (a) is killed.

2. A measurement method for complement-dependent bactericidal activity according to claim 1, wherein the certain amount in the step 3) is 50%.

3. A measurement method for complement-dependent bactericidal activity according to claim 1 or 2, wherein a usage amount of the complement component (c) in the step 1) is from 1/400 to 1/100 with respect to that of a complement component (c') to be used in a case of measurement using encapsulated *Streptococcus pneumoniae* (a').

4. A measurement method for complement-dependent bactericidal activity according to any one of claims 1 to 3, wherein the capsule-deficient *Streptococcus pneumoniae* as defined in each of the steps 1) and 2) comprises non-encapsulated *Streptococcus pneumoniae* or transparent *Streptococcus pneumoniae.*

5. A measurement method according to any one of claims 1 to 4, wherein the capsule-deficient *Streptococcus pneumoniae* (a) comprises capsule-deficient *Streptococcus pneumoniae* derived from any one selected from pneumococcal strains WU2, D39, KK1162, ATCC6303, BG9739, TIGR4, and EF5668.

6. A measurement method according to any one of claims 1 to 5, wherein the phagocyte (d) in the step 1) comprises an HL-60-derived cell.

7. A measurement method according to any one of claims 1 to 6, wherein the sample (b) comprises a blood sample collected from a test subject.

8. A measurement method according to claim 7, wherein the sample (b) comprises a blood sample collected from a test subject vaccinated with a pneumococcal vaccine.

9. An examination method for immunogenicity of a pneumococcal vaccine, the examination method comprising performing an examination based on a result measured by the measurement method of any one of claims 1 to 8.


SEQUENCE LISTING

<110>  THE RESEARCH FOUNDATION FOR MICROBIAL DISEASES OF OSAKA UNIVERSITY
       OSAKA UNIVERSITY

<120>  Pneumococcal Opsonophagocytic Killing Assay

<130>  GP16-1010PCT

<150>  JP P2015-122520
<151>  2015-06-18

<160>  4

<170>  PatentIn version 3.1

<210>  1
<211>  29
<212>  DNA
<213>  Artificial

<220>
<223>  Primer for PsaA-F(NdeI)

<400>  1
ggaattccat atggctagcg gaaaaaaag                                        29


<210>  2
<211>  29
<212>  DNA
<213>  Artificial

<220>
<223>  Primer for PsaA-R(HingIII)

<400>  2
cccaagcttt tattttgcca atccttcag                                        29


<210>  3
<211>  36
<212>  DNA
<213>  Artificial

<220>
<223>  Primer for CbpA-F(NdeI)

<400>  3
ggaattccat atgacagaga acgagggaag taccca                                36


<210>  4
<211>  34
<212>  DNA
<213>  Artificial

<220>
<223>  Primer for CbpA-R(HingIII)

<400>  4
cccaagcttt taccacatac cgttttcttg tttc                                  34